(19) Europäisches Patentamt
European Patent Office
Office européen des brevets

(11) Veröffentlichungsnummer: 0 070 331
A2

(12) # EUROPÄISCHE PATENTANMELDUNG

(21) Anmeldenummer: 81106901.2

(22) Anmeldetag: 03.09.81

(51) Int. Cl.³: C 07 D 501/36, A 61 K 31/545

(30) Priorität: 17.07.81 US 284160
04.08.81 CH 5018/81

(43) Veröffentlichungstag der Anmeldung: 26.01.83
Patentblatt 83/4

(84) Benannte Vertragsstaaten: AT BE CH DE FR IT LI LU NL SE

(71) Anmelder: F. HOFFMANN-LA ROCHE & CO.
Aktiengesellschaft, CH-4002 Basel (CH)

(72) Erfinder: Montavon, Marc, Dr., Realpstrasse 72,
CH-4054 Basel (CH)
Erfinder: Reiner, Roland, Dr., Rheinfelderstrasse 8,
CH-4058 Basel (CH)

(74) Vertreter: Lederer, Franz, Dr. et al, Patentanwälte Dr.
Franz Lederer Reiner F. Meyer Lucile-Grahn-Strasse 22,
D-8000 München 80 (DE)

(54) **Cephalosporinderivate, entsprechende Präparate, die Verwendung dieser Produkte bei der Behandlung von Krankheiten sowie die Herstellung der Werkstoffe.**

(57) Neue Cephalosporinderivate der allgemeinen Formel

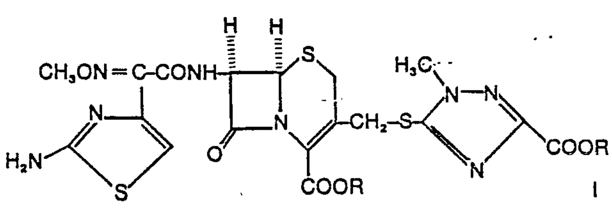

I

in der R die Pivaloyloxymethyl- oder Acetoxymethylgruppe darstellt,
sowie Säureadditionssalze dieser Verbindungen und Hydrate der Verbindungen der Formel I bzw. von deren Säureadditionssalzen, deren Verwendung bei der Behandlung von Infektionskrankheiten, deren Herstellung sowie dabei verwendbare Ausgangsverbindungen.

EP 0 070 331 A2

0070331

F.Hoffmann-La Roche & Co.Aktiengesellschaft,Basel,Schweiz

3. Sep. 1981
RAN 4410/156

Cephalosporinderivate,
entsprechende Präparate, die Verwendung dieser Produkte bei der Behandlung von Krankheiten sowie die Herstellung der Wirkstoffe.

Die vorliegende Erfindung betrifft neue Cephalosporinderivate der allgemeinen Formel

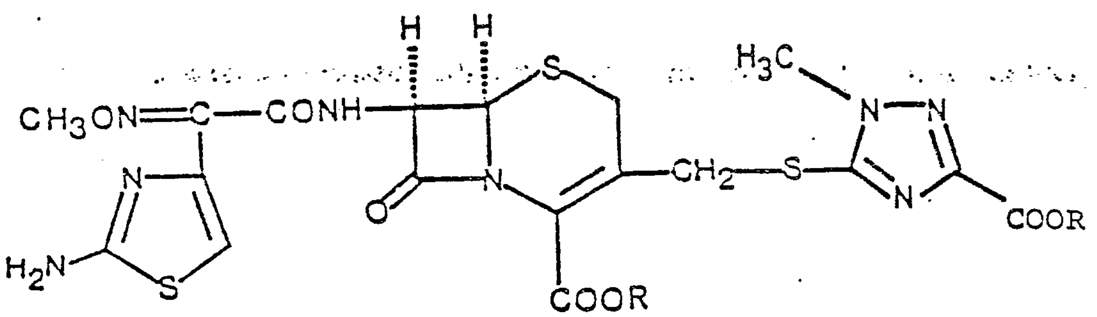

I

in der R die Pivaloyloxymethyl- oder Acetoxymethylgruppe darstellt,

sowie Säureadditionssalze dieser Verbindungen und Hydrate der Verbindungen der Formel I bzw. von deren Säureadditionssalzen.

Die Verbindungen der Formel I bilden Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze,

Mn/ 13.8.81

wie Sulfate, Nitrate, Phosphate und dgl., Alkyl- und Monoaryl-sulfonate, wie Aethansulfonate, Toluolsulfonate, Benzolsulfonate und dgl., und auch andere organische Säuresalze, wie Acetate, Tartrate, Maleate, Citrate, Benzoate, Salicylate, Ascorbate und dgl.

Die Verbindungen der Formel I sowie deren Säureadditionssalze können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der syn-isomeren Form

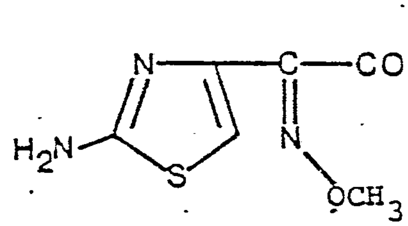

oder in der anti-isomeren Form

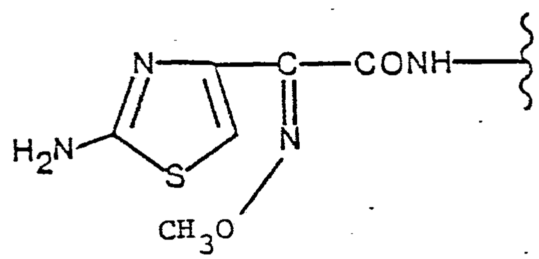

bzw. als Gemische dieser beiden Formen vorliegen. Bevorzugt ist die syn-isomere Form bzw. Gemische, in denen die syn-isomere Form überwiegt.

Bevorzugte Produkte sind

- Methylen(6R,7R)-7-[2-(2-Amino-4-thiazolyl)-2-[(Z)-methoxyimino]-acetamido]-3-[[[2-methyl-5-[[(pivaloyloxy)-methoxy]carbonyl]-2H-1,2,4-triazol-3-yl]thio]methyl]-8-oxo-5-thia-1-azabicyclo[4.2.0]oct-2-en-2-carboxylat-pivalat,
und dessen Säureadditionssalze sowie die entsprechenden Hydrate.

Die obigen Cephalosporinderivate können erfindungsgemäss dadurch hergestellt werden, dass man die entsprechende Dicarbonsäure, d.h. die (6R,7R)-7-[2-(2-Amino-4-thiazolyl)-2-[methoxyimino]acetamido]-3-[[3-carboxy-1-methyl-1H-1,2,4-triazol-5-yl)thio]methyl]-8-oxo-5-thia-1-azabicyclo[4.2.0]oct-2-en-2-carbonsäure (Verbindung II), oder ein Salz dieser Verbindung einer entsprechenden Veresterung unterwirft und erwünschtenfalls das erhaltene Produkt in ein Säureadditionssalz oder Hydrat bzw. ein Hydrat dieses Säureadditionssalzes überführt.

Die im obigen Verfahren verwendete Dicarbonsäure (Verbindung II) kann ausgehend von 7-Aminocephalosporansäure wie folgt hergestellt werden:

Die 7-Aminocephalosporansäure wird in wässriger Lösung mit 4,5-Dihydro-1-methyl-5-thioxo-1H-1,2,4-triazol-3-carbonsäure (Verbindung III) umgesetzt, z.B. bei einer Temperatur zwischen etwa 40 und 80°C, zweckmässig bei etwa 60°C. Die Lösung wird vorzugsweise bei einem pH von etwa 6 bis 7, vorzugsweise 6,5, gepuffert.

Die so erhaltene (6R,7R)-7-Amino-3-/[(3-carboxy-1-methyl-1,2,4-triazol-5-yl)thio]methyl/-8-oxo-5-thia-1-azabicyclo[4.2.0]oct-2-en-2-carbonsäure (Verbindung IV) wird anschliessend mit einer Carbonsäure der allgemeinen Formel

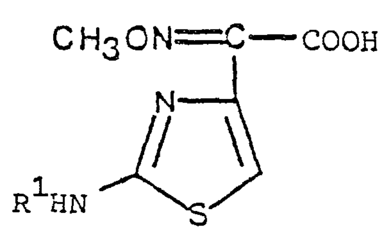

V

worin $R^1$ eine abspaltbare Gruppe darstellt, oder mit einem reaktionsfähigen funktionellen Derivat dieser Säure umgesetzt, wobei man eine Verbindung der allgemeinen Formel

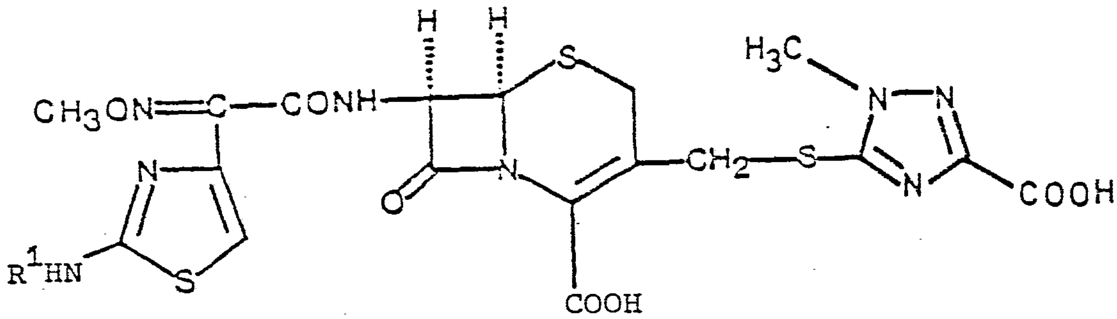

VI

worin $R^1$ die obige Bedeutung hat, erhält.

Mögliche $R^1$-Schutzgruppen sind beispielsweise sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Bevorzugte $R^1$-Schutzgruppen sind Chlor-, Brom- und Jodacetyl, insbesondere Chloracetyl. Letztere Schutzgruppen können durch Behandeln mit Thioharnstoff abgespalten werden.

Als reaktionsfähige funktionelle Derivate von Carbonsäuren der Formel V kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung IV mit der Carbonsäure der Formel V oder einem reaktionsfähigen funktionellen Derivat davon kann in an sich bekannter Weise durchgeführt werden. So kann man z.B. ein Säurehalogenid, vorzugsweise das Chlorid einer Carbonsäure der Formel V mit dem

Amin IV umsetzen. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, ggfs. im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Die Umsetzung kann zweckmässig bei Temperaturen zwischen etwa -40°C und Zimmertemperatur, beispielsweise bei etwa 0-10°C, erfolgen.

Anschliessend wird in der erhaltenen Verbindung der Formel VI die Aminoschutzgruppe $R^1$ abgespalten, wobei die gewünschte Dicarbonsäure (Verbindung II) erhalten wird. Durch saure Hydrolyse abspaltbare Schutzgruppen werden vorzugsweise mit Hilfe einer niederen Alkancarbonsäure, die ggfs. halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa 0°C bis +40°C. Alkalisch abspaltbare Schutzgruppen werden im allgemeinen mit verdünnter wässriger Lauge bei 0°C bis 30°C hydrolysiert. Die Chloracetyl-, Bromacetyl- und Jodacetyl-Schutzgruppen können mittels Thioharnstoff in saurem, neutralem oder alkalischem Milieu bei etwa 0-30°C abgespalten werden. Hydrogenolytische Abspaltung (z.B. Abspaltung von Benzyl) ist hier ungeeignet, da bei der Hydrogenolyse die Oximfunktion zur Aminogruppe reduziert würde.

Die erhaltene Dicarbonsäure II kann anschliessend in ein Salz übergeführt werden, z.B. in ein Säureadditionssalz wie oben für die Endprodukte der Formel I beschrieben, oder auch in Salze mit Basen, z.B. Alkalimetallsalze, wie das

Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin. Die Salze können Monosalze oder auch Disalze sein. Die Salze werden in an sich bekannter Weise hergestellt, z.B. durch Umsetzung der Dicarbonsäure II mit einer äquivalenten Menge der gewünschten Säure bzw. Base, zweckmässig in einem Lösungsmittel wie Wasser oder in einem organischen Lösungsmittel, wie Aethanol Methanol, Aceton und anderem mehr. Bei Verwendung eines zweiten Aequivalents an Base erfolgt Salzbildung auch an der zweiten Carboxyfunktion. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die 4,5-Dihydro-1-methyl-5-thioxo-1H-1,2,4-triazol-3-carbonsäure steht in tautomerem Gleichgewicht mit dem entsprechenden Thiol im Sinne der nachstehenden Formeln

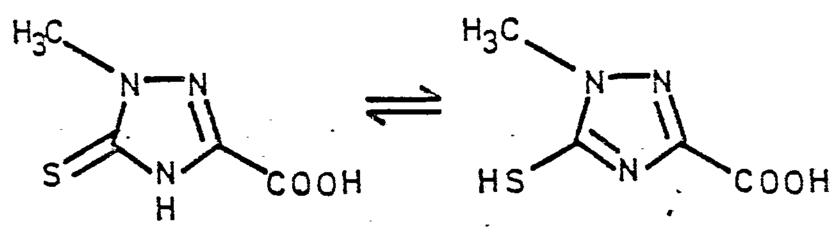

III III a

Die Herstellung dieser Verbindung wird in Beispiel 1 beschrieben.

Die erfindungsgemässe Veresterung der Dicarbonsäure II erfolgt durch Umsetzung mit einem die Pivaloyloxymethyl- oder Acetoxymethylgruppe abgebenden Mittel, vorzugsweise durch Umsetzung mit dem entsprechenden Halogenid, insbesondere mit dem Jodid. Die Reaktion kann mit Hilfe einer Base, z.B. einem Alkalimetallhydroxid oder -carbonat, oder

einem organischen Amin, wie Triäthylamin, beschleunigt werden. Vorzugsweise verwendet man einen Ueberschuss an dem entsprechenden Halogenid. Die Veresterungsreaktion wird vorzugsweise in einem inerten organischen Lösungsmittel, ggfs. im Gemisch mit Wasser, durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0-40°C.

Die Herstellung der Säureadditionssalze und Hydrate der Verbindungen der Formel I bzw. der Hydrate dieser Salze kann in an sich bekannter Weise erfolgen, die Säureadditionssalze z.B. durch Umsetzung der Verbindung der Formel I mit einer äquivalenten Menge der gewünschten Säure, zweckmässig in einem Lösungsmittel wie Wasser oder in einem organischen Lösungsmittel, wie Aethanol, Methanol, Aceton und anderen mehr. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt einer feuchten Atmosphäre, z.B. bei etwa +10°C bis +40°C, ausgesetzt werden.

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches. Vorzugsweise erhält man aber die Verbindung der Formel I in der syn-isomeren Form, indem man eine Ausgangsverbindung der Formel V in syn-Form einsetzt.

Die Verbindungen der Formel I sowie die entsprechenden Säureadditionssalze bzw. die Hydrate dieser Produkte sind antibiotisch, insbesondere bakterizid wirksam. Sie besitzen ein breites Wirkungsspektrum gegen Gram-positive Bakterien, z.B. Streptokokken, und gegen Gram-negative Bakterien, wie z.B. Neisseria meningitidis, sowie gegen verschiedene β-Lactamase-bildende Gram-negative Erreger, wie Escherichia coli und Serratia marcescens.

Die Verbindungen der Formel I sowie die entsprechenden Säureadditionssalze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 4 g, insbesondere etwa 0,25 g bis etwa 2 g, in Betracht. Enterale oder parenterale Verabreichung ist möglich, ein besonderer Vorzug liegt in der Verwendbarkeit der erfindungsgemässen Produkte für die enterale, z.B. die orale, Verabreichung.

Die orale antimikrobielle Wirksamkeit der erfindungsgemässen Produkte kann anhand von in vivo-Versuchen an der Maus nachgewiesen werden. Im nachstehenden bedeutet die kurative Dosis ($CD_{50}$, mg/kg) diejenige Dosis, bei der 50% der getesteten Mäuse überleben.

Testverbindungen:

| | |
|---|---|
| Produkt A: | Methylen (6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat |
| Cephalexin | (vorbekanntes, anerkannt oral wirksames Cephalosporin) |

Ergebnis ($CD_{50}$, mg/kg)

| Erreger | Produkt A p.o. | Produkt A s.c. | Cephalexin p.o. | Cephalexin s.c. |
|---|---|---|---|---|
| Escherichia coli | 0,047 | 0,002 | 3,2 | 2,1 |
| Serratia marcescens | 0,68 | 0,05 | > 50 | > 50 |
| Neisseria meningitidis | 0,14 | | 1,2 | |
| Streptococcus pyogenes | 0,95 | 0,044 | 1,8 | 1,2 |

Das Produkt A ist bedeutend wirksamer als Cephalexin sowohl nach peroraler als auch nach subcutaner Gabe.

Die nach oraler Gabe erzielten Blutspiegelwerte ergeben sich gemäss folgendem Vergleich an der Ratte.

| | orale Dosis | Antibiotikumkonzentration im Blut in µg/ml nach Minuten | | | | | |
|---|---|---|---|---|---|---|---|
| | mg/kg | 15 | 30 | 45 | 60 | 90 | 150 |
| Produkt A | 20 | 6 | 12 | 12 | 10 | 8 | 5 |
| Cephalexin | 50 | 8 | 12 | 14 | 18 | | 5 |

Das Produkt A ist mindestens so ungiftig wie Cephalexin ($LD_{50}$ p.o. an der Maus nach 24 Stunden: > 4000 mg/kg für Produkt A, 1600-4500 mg/kg für Cephalexin).

Die erfindungsgemässen Produkte können als Heilmittel, z.B. in Form pharmazeutischer Präparate Verwendung finden, welche sie oder ihre Salze in Mischung mit einem für die enterale oder parenterale, bevorzugt enterale, Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester

Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutisch wertvolle Stoffe enthalten.

Beispiel 1

Herstellung von Methylen (6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]-thio]methyl]-8-oxo-5-thia-1-azabicyclo[4.2.0]oct-2-en-2-carboxylat-pivalat:

3,9 g des Dinatriumsalzes der (6R,7R)-7-[2-(2-Amino-4-thiazolyl)-2-[(Z)-methoxyimino]acetamido]-3-[[3-carboxy-1-methyl-1H-1,2,4-triazol-5-yl)thio]methyl]-8-oxo-5-thia-1-azabicyclo[4.2.0]oct-2-en-2-carbonsäure werden in einem Gemisch von 100 ml Dimethylformamid und 25 ml Wasser gelöst. Diese Lösung wird bei 0-5°C unter Stickstoff-Begasung mit 3,13 g Jodmethylpivalat versetzt. Das Reaktionsgemisch wird 30 Minuten bei 0-5°C gerührt, danach auf 500 ml Wasser gegossen und dreimal mit je 200 ml Aethylacetat extrahiert. Die wässrige Phase wird verworfen. Die vereinigten Aethylacetatphasen werden nacheinander mit Wasser, verdünnter wässriger Natriumhydrogencarbonatlösung und nochmals mit Wasser gewaschen, über Natriumsulfat getrocknet und im Vakuum bei 40°C stark eingeengt. Nach Zugabe von tiefsiedendem Petroläther wird das ausgefallene Rohprodukt abgenutscht, mit tiefsiedendem Petroläther gewaschen und im Hochvakuum bei 40°C getrocknet. Man erhält beigefarbenes, amorphes Rohprodukt. Zwecks Reinigung wird dieses Produkt an einer Säule mit 100 g Kieselgel (0,2-0,5 mm) und Aethylacetat als Elutionsmittel chromatographiert. Man erhält reine, beigefarbene, amorphe Titelsubstanz mit Rf = 0,31 in der Dünnschichtchromatographie auf Kieselgel-60F-254-Fertigplatten mit Aethylacetat als Laufmittel. Die spezifische Drehung beträgt $[\alpha]_D^{25}$ = -121,6° (c = 0,4924 in Aceton); das Kernresonanzspektrum und die Mikroanalyse entsprechen der angegebenen Struktur.

Die im obigen Verfahren als Ausgangsmaterial verwendete Verbindung kann wie folgt hergestellt werden:

a) Herstellung von 4,5-Dihydro-1-methyl-5-thioxo-1H-1,2,4-triazol-3-carbonsäure-methylester:

12,9 g 1-Methoxyoxalyl-2-methyl-thiosemicarbazid werden einer Lösung von 1,6 g Natrium in 200 ml Methanol zugegeben. Das Gemisch wird 4 Stunden unter Rückfluss gekocht. Die nach einer 1/2 Stunde ausgefallene Substanz wird abgetrennt. Die Mutterlauge wird im Vakuum bei 40°C eingedampft. Der Eindampfrückstand wird in 100 ml Wasser gelöst und mit konz. Salzsäure angesäuert. Das dabei ausgeschiedene Kristallisat liefert nach dem Umkristallisieren aus Wasser reinen, farblosen 4,5-Dihydro-1-methyl-5-thioxo-1H-1,2,4-thiazol-3-carbonsäure-methylester vom Schmelzpunkt 188-190°C (Zers.).

b) Herstellung von 4,5-Dihydro-1-methyl-5-thioxo-1H-1,2,4-triazol-3-carbonsäure:

3,46 g 4,5-Dihydro-1-methyl-5-thioxo-1H-1,2,4-triazol-3-carbonsäure-methylester werden in 50 ml 1N Natronlauge gelöst. Nach halbstündigem Rühren bei 25°C wird die Reaktionslösung mit 25 ml 2N Salzsäure sauer gestellt und im Vakuum bei 40°C eingeengt, wobei die gewünschte Säure kristallisiert. Diese wird abgenutscht, mit Eiswasser gewaschen und im Hochvakuum bei 45°C getrocknet. Man erhält farblose 4,5-Dihydro-1-methyl-5-thioxo-1H-1,2,4-triazol-3-carbonsäure vom Schmelzpunkt 177-178°C (Zers.).

c) Herstellung von (6R,7R)-7-Amino-3-[[(3-carboxy-1-methyl-1,2,4-triazol-5-yl)thio]methyl]-8-oxo-5-thia-1-azabicyclo[4.2.0]oct-2-en-2-carbonsäure:

34 g 7-Aminocephalosporansäure werden zusammen mit 25,5 g 4,5-Dihydro-1-methyl-5-thioxo-1H-1,2,4-triazol-3-carbonsäure in 500 ml Wasser suspendiert. Das pH wird mit 3N Natronlauge auf 6,5 gestellt. Das Gemisch wird 4 Stunden bei 55°C unter Stickstoffbegasung gerührt, wobei das pH mit 3N Natronlauge mit Hilfe eines Autotitrators konstant

bei 6,5 gehalten wird. Die dunkle Lösung wird auf 25°C gekühlt und mit konz. Salzsäure auf pH 3,5 gestellt. Nach 15 Minuten wird das ausgefallene Material abgenutscht, in 500 ml Wasser suspendiert und mit 3N Natronlauge bei pH 7 in Lösung gebracht. Die orangerote Lösung wird mit konz. Salzsäure auf pH 3 gestellt. Die ausgefallene Substanz wird abgenutscht und nacheinander mit 250 ml Wasser, 500 ml Aceton, 500 ml tiefsiedendem Petroläther gewaschen und über Nacht im Vakuum bei 40-45°C getrocknet. Man erhält (6R,7R)-7-Amino-3-[[(3-carboxy-1-methyl-1,2,4-triazol-5-yl)thio]methyl]-8-oxo-5-thia-1-azabicyclo[4.2.0]oct-2-en-2-carbonsäure als beige gefärbtes Pulver.

d) Herstellung von (6R,7R)-3-[[(3-Carboxy-1-methyl-1H-1,2,4-triazol-5-yl)thio]methyl]-7-[2-[2-(2-chloracetamido)-4-thiazolyl]-2-[(Z)-methoxyimino]acetamido]-8-oxo-5-thia-1-azabicyclo[4.2.0]oct-2-en-2-carbonsäure:

Zu 600 ml über Calciumchlorid getrocknetem Methylenchlorid werden 75 g Phosphorpentachlorid gegeben. Die Suspension wird unter Rühren auf -10°C abgekühlt, und während ca. 5 Minuten werden 138 ml N,N-Dimethylacetamid zugetropft, wobei die Temperatur auf -5°C ansteigt. Die Suspension wird auf -15°C gekühlt und 15 Minuten gerührt. Nach dem Abkühlen auf -20°C werden 100 g 2-(2-Chloracetamido-4-thiazolyl)-2-(Z)-methoxyimino-essigsäure zugegeben und 45 Minuten bei -15°C gerührt, wobei eine gelb-orange Lösung entsteht. Diese Lösung wird auf -30°C gekühlt, mit 150 g Eis versetzt und 10 Minuten bei -5°C gerührt. Die das gebildete Säurechlorid enthaltende Methylenchlorid-Phase wird abgetrennt und zunächst bei ca. -15°C aufbewahrt. Diese Säurechlorid-Lösung wird einer auf 0-5°C gekühlten Lösung, die durch Versetzen von 111,5 g (6R,7R)-7-Amino-3-[[(3-carboxy-1-methyl-1,2,4-triazol-5-yl)thio]methyl]-8-oxo-5-thia-1-azabicyclo[4.2.0]oct-2-en-carbonsäure mit 3N Natronlauge bis pH 8,2 in 500 ml Wasser hergestellt wurde, während 30 Minuten unter kräftigem Rühren zugetropft, wobei der pH-Wert mit 3N Natronlauge mit Hilfe eines

Autotitrators bei 8,0-8,2 gehalten wird. Das Gemisch wird 2 Stunden bei 25°C gerührt. Danach werden 900 ml Methylenchlorid und 6 l n-Butanol zugegeben. Unter kräftigem Rühren wird das pH mit 3N Salzsäure auf 2 zurückgestellt. Die organische Phase wird abgetrennt, dreimal mit je 3 l Wasser gewaschen, mit 60 g Entfärbungskohle 15 Minuten lang verrührt und filtriert. Das hellgelbe Filtrat wird im Vakuum bei 60°C stark eingeengt, wobei das Reaktionsprodukt ausfällt. Letzteres wird abgenutscht und nacheinander mit n-Butanol, Aether und tiefsiedendem Petroläther gewaschen und über Nacht im Vakuum bei 40°C getrocknet. Man erhält rohe, beige (6R,7R)-3-[[(3-Carboxy-1-methyl-1H-1,2,4-triazol-5-yl)thio]methyl]-7-[2-(2-chloracetamido)-4-thiazolyl]-2-[(Z)-methoxyimino]acetamido]-8-oxo-5-thia-1-azabicyclo[4.2.0]oct-2-en-2-carbonsäure.

e) Herstellung des Dinatriumsalzes der (6R,7R)-7-[2-(2-Amino-4-thiazolyl)-2-[(Z)-methoxyimino]acetamido]-3-[[(3-carboxy-1-methyl-1H-1,2,4-triazol-5-yl)thio]-methyl]-8-oxo-5-thia-1-azabicyclo[4.2.0]oct-2-en-2-carbonsäure:

53 g (6R,7R)-3-[[(3-Carboxy-1-methyl-1H-1,2,4-triazol-5-yl)thio]methyl]-7-[2-[2-(2-chloracetamido)-4-thiazolyl)-2-[(Z)-methoxyimino]acetamido]-8-oxo-5-thia-1-azabicyclo-[4.2.0]oct-2-en-2-carbonsäure werden in 1 l Wasser zusammen mit 34 g Thioharnstoff suspendiert. Das pH wird durch Zutropfen von 1N Natronlauge auf 7 gestellt, wobei eine dunkelbraune Lösung entsteht. Diese wird über Nacht unter Stickstoffbegasung bei pH 7 gerührt, wobei das pH mit 1N Natronlauge mit Hilfe eines Autotitrators konstant gehalten wird. Das pH der Reaktionslösung wird mit 1N Salzsäure auf 3,5 unter Rühren zurückgestellt. Die ausgefallene Substanz (Fraktion I) wird abgenutscht, mit 250 ml Wasser gewaschen und im Vakuum bei 40°C getrocknet. Die Fraktion I ist braun gefärbt und stark verunreinigt; sie wird verworfen. Die orange Mutterlauge wird mit 1N Salzsäure unter Rühren auf pH 2,65 zurückgestellt. Das ausgefallene Material wird abgenutscht und mit 250 ml Wasser gewaschen. Das beige-farbene Nutschgut wird unter vermindertem Druck mit Aethanol azeotropiert, abgenutscht, mit Aethanol und tiefsiedendem Petroläther gewaschen und im Vakuum bei 40°C getrocknet. Man erhält so eine beigebraune Fraktion II, welche zwecks Reinigung und Ueberführung in das Dinatriumsalz in 2 l Methanol suspendiert, mit 50 ml einer 2N Lösung des Natriumsalzes der 2-Aethylcapronsäure in Aethylacetat versetzt und anschliessend mit 200 ml Wasser 30 Minuten gerührt wird. Wenig ungelöstes, braunes Material wird abfiltriert und verworfen. Vom orange gefärbten Filtrat wird im Vakuum bei 40°C ca. 1 l abgedampft, dann 1 l Aethanol zugegeben und im Vakuum auf ein Volumen von ca. 500 ml eingeengt. Vom dabei ausgefallenen, schmierigen braunen Harz, welches ein starkes Gemisch darstellt, wird abdekantiert. Die abdekantierte gelborange Lösung wird mit 1 l Aethanol versetzt und im Vakuum bei 40°C stark eingeengt, wobei teilweise Substanz ausfällt. Nach Zugabe von 2,5 l Methanol entsteht eine gelbe Lösung, welche im Vakuum bei 40°C stark eingeengt wird. Das dabei ausgefallene Dinatriumsalz wird abgenutscht, mit Methanol und tiefsiedendem Petroläther gewaschen und im Hochvakuum bei 35°C getrocknet. Man erhält reines, beige-farbenes Dinatriumsalz der (6R,7R)-7-[2-(2-Amino-4-thiazolyl)-2-[(Z)-methoxyimino]acetamido]-3-[[(3-carboxy-1-methyl-1H-1,2,4-triazol-5-yl)thio]methyl]-8-oxo-5-thia-1-azabicyclo[4.2.0]oct-2-en-2-carbonsäure mit $[\alpha]_D^{25} = -38,7°$ (c = 1 in Wasser).

## Beispiel 2

Herstellung von Methylen (6R,7R)-7-[2-(2-amino-4-thiazolyl)-2-[(Z)-methoxyimino]-acetamido]-3-[[[2-methyl-5-[[(acetoxy)methoxy]carbonyl]-2H-1,2,4-triazol-3-yl]-thio]methyl]-8-oxo-5-thia-1-azabicyclo[4.2.0]oct-2-en-2-carboxylat-acetat:

5,98 g (6R,7R)-7-Amino-3-[[(3-carboxy-1-methyl-1,2,4-triazol-5-yl)thio]methyl]-8-oxo-5-thia-1-azabicyclo[4.2.0]-oct-2-en-2-carbonsäure-Dinatriumsalz werden in einem Gemisch von 70 ml Dimethylformamid und 25 ml Wasser gelöst. Die Lösung wird unter Stickstoff-Begasung bei 0-5°C mit 6,12 g Essigsäure-brommethylester versetzt und 3 Stunden bei 0-5°C gerührt. Das Reaktionsgemisch wird auf 500 ml Wasser gegossen und zweimal mit Aethylacetat unter Zusatz von wenig Aceton extrahiert. Die vereinigten organischen Phasen werden nacheinander mit verdünnter wässriger Natriumhydrogencarbonat-Lösung und Wasser gewaschen, über Natriumsulfat getrocknet und im Vakuum bei 40°C stark eingeengt. Nach Zugabe von tiefsiedendem Petroläther wird die amorph ausgefallene Substanz abgenutscht, mit tiefsiedendem Petroläther gewaschen und über Nacht im Hochvakuum bei 25°C getrocknet. Man erhält ein beiges Rohprodukt, welches, zwecks Reinigung, an einer Säule mit 80 g Kieselgel (0,2-0,5 mm Durchmesser) und Aethylacetat als Elutionsmittel chromatographiert wird. Man erhält reine beige, amorphe Titelsubstanz (aus Aethylacetat/tiefsiedendem Petroläther) mit $[\alpha]_D^{25}$ = -107,16° (c = 1,0358 in Aceton). Das Kernresonanzspektrum und die Mikroanalyse entsprechen der angegebenen Struktur.

## Beispiel 3

Es wird in üblicher Weise eine Gelatine-Steckkapsel folgender Zusammensetzung hergestellt:

| | |
|---|---|
| Methylen (6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

0070331

## Beispiel 4

Es wird in üblicher Weise eine Tablette folgender Zusammensetzung hergestellt:

| | |
|---|---|
| Methylen (6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat | 250 mg |
| Lactose | 70 mg |
| Maisstärke | 65 mg |
| Polyvinylpyrrolidon | 10 mg |
| Magnesiumstearat | 5 mg |
| | 400 mg |

Mit dem Wirkstoff, der Lactose, dem Polyvinylpyrrolidon und 40 Gewichtsteilen der Maisstärke wird in üblicher Weise ein Granulat hergestellt. Dieses wird mit den restlichen 25 Gewichtsteilen Maisstärke und 5 Gewichtsteilen Magnesiumstearat vermischt und zu Tabletten gepresst.

Patentansprüche

1. Cephalosporinderivate der allgemeinen Formel

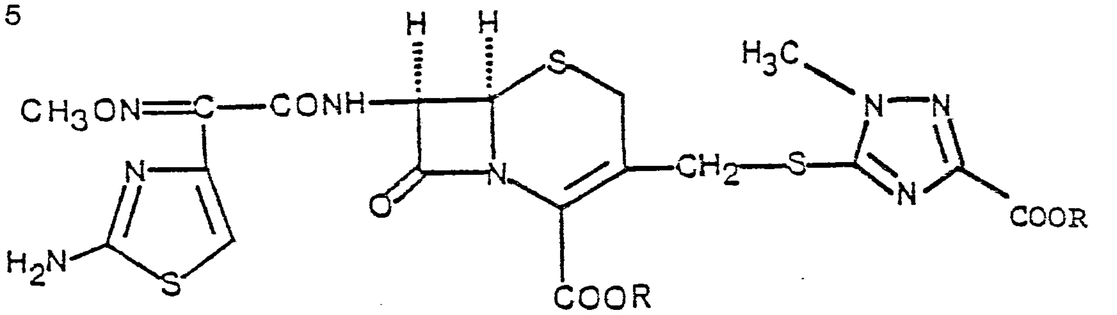

I

in der R die Pivaloyloxymethyl- oder Acetoxymethylgruppe darstellt, sowie Säureadditionssalze dieser Verbindungen und Hydrate der Verbindungen der Formel I bzw. von deren Säureadditionssalzen.

2. Cephalosporinderivate gemäss Anspruch 1 in der syn-isomeren Form bzw. Gemische, in denen die syn-isomere Form überwiegt.

3. Methylen (6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionssalze.

4. Methylen (6R,7R)-7-[2-(2-Amino-4-thiazolyl)-2-[(Z)-methoxyimino]-acetamido]-3-[[[2-methyl-5-[[(acetoxy)-methoxy]carbonyl]-2H-1,2,4-triazol-3-yl]thio]methyl]-8-oxo-5-thia-1-azabicyclo[4.2.0]oct-2-en-2-carboxylat-acetat sowie Säureadditionssalze dieser Verbindung und Hydrate dieser Verbindung bzw. Säureadditionssalze.

5. Verbindungen gemäss einem der Ansprüche 1-4 als

pharmazeutische Wirkstoffe.

6. Verbindungen gemäss einem der Ansprüche 1-4 als pharmazeutische Wirkstoffe zur enteralen, z.B. oralen, Behandlung und Prophylaxe von Infektionskrankheiten.

7. Methylen (6R,7R)-7-[2-(2-Amino-4-thiazolyl)-2-[(Z)-methoxyimino]-acetamido]-3-[[[2-methyl-5-[[(pivaloyloxy)methoxy]carbonyl]-2H-1,2,4-triazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionssalze zur enteralen, z.B. oralen, Behandlung und Prophylaxe von Infektionskrankheiten.

8. Pharmazeutische Präparate, gekennzeichnet durch einen Gehalt an einer Verbindung gemäss einem der Ansprüche 1-4.

9. Pharmazeutische Präparate zur enteralen, z.B. oralen, Behandlung und Prophylaxe von Infektionskrankheiten, gekennzeichnet durch einen Gehalt an einer Verbindung gemäss einem der Ansprüche 1-4.

10. Pharmazeutische Präparate gemäss Anspruch 9 enthaltend Methylen (6R,7R)-7-[2-(2-Amino-4-thiazolyl)-2-[(Z)-methoxyimino]-acetamido[-3-[[[2-methyl-5-[[(pivaloyloxy)methoxy]carbonyl]-2H-1,2,4-triazol-3-yl]thio]-methyl]-8-oxo-5-thia-1-azabicyclo[4.2.0]oct-2-en-2-carboxylat-pivalat oder ein Säureadditionssalz dieser Verbindung bzw. ein Hydrat dieser Verbindung bzw. eines seiner Säureadditionssalze.

11. Verfahren zur Herstellung der Verbindungen gemäss einem der Ansprüche 1-4, dadurch gekennzeichnet, dass man die entsprechende Dicarbonsäure, d.h. die (6R,7R)-7-[2-(2-Amino-4-thiazolyl)-2-[methoxyimino]acetamido]-3-[[3-carboxy-1-methyl-1H-1,2,4-triazol-5-yl)thia]-

methyl]-8-oxo-5-thia-1-azabicyclo[4.2.0]oct-2-en-2-carbonsäure oder ein Salz dieser Verbindung einer entsprechenden Veresterung unterwirft und erwünschtenfalls das erhaltene Produkt in ein Säureadditionssalz oder Hydrat bzw. ein Hydrat dieses Säureadditionssalzes überführt.

12. Verfahren nach Anspruch 11, dadurch gekennzeichnet, dass man als veresterndes Mittel ein Pivaloyloxymethylhalogenid, insbesondere das Jodid, verwendet.

Patentansprüche
OESTERREICH.

1. Verfahren zur Herstellung von Cephalosporinderivaten der allgemeinen Formel

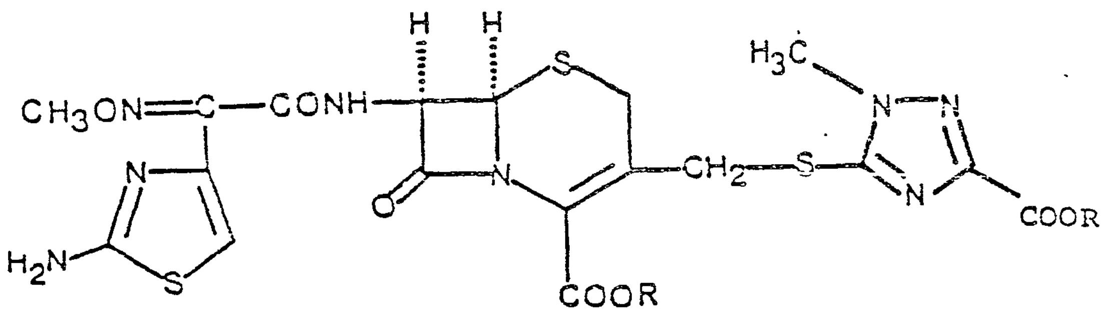

I

in der R die Pivaloyloxymethyl- oder Acetoxymethylgruppe darstellt,

sowie von Säureadditionssalzen dieser Verbindungen und von Hydraten der Verbindungen der Formel I bzw. von deren Säureadditionssalzen, dadurch gekennzeichnet, dass man die entsprechende Dicarbonsäure, d.h. die (6R,7R)-7-[2-(2-Amino-4-thiazolyl)-2-[methoxyimino]acetamido]-3-[[3-carboxy-1-methyl-1H-1,2,4-triazol-5-yl)thia]methyl]-8-oxo-5-thia-1-azabicyclo[4.2.0]oct-2-en-2-carbonsäure oder ein Salz dieser Verbindung einer entsprechenden Veresterung unterwirft und erwünschtenfalls das erhaltene Produkt in ein Säureadditionssalz oder Hydrat bzw. ein Hydrat dieses Säureadditionssalzes überführt.

2. Verfahren nach Anspruch 1 zur Herstellung von Cephalosporinderivaten gemäss Anspruch 1 in der syn-isomeren Form bzw. von Gemischen, in denen die syn-isomere Form überwiegt, dadurch gekennzeichnet, dass man eine Ausgangsverbindung mit entsprechender Konfiguration einsetzt oder ein erhaltenes syn/anti-Gemisch auftrennt.

3. Verfahren nach Anspruch 2 zur Herstellung von Methylen (6R,7R)-7-[2-(2-Amino-4-thiazolyl)-2-[(Z)-methoxyimino]-acetamido]-3-[[[2-methyl-5-[[(pivaloyloxy)methoxy]-carbonyl]-2H-1,2,4-triazol-3-yl]thio]methyl]-8-oxo-5-thia-1-azabicyclo[4.2.0]oct-2-en-2-carboxylat-pivalat sowie

von Säureadditionssalzen dieser Verbindung und von Hydraten dieser Verbindung bzw. Säureadditionssalze, dadurch gekennzeichnet, dass man ein entsprechendes Veresterungsmittel einsetzt.

4. Verfahren nach Anspruch 2 zur Herstellung von Methylen (6R,7R)-7-[2-(2-Amino-4-thiazolyl)-2-[(Z)-methoxyimino]-acetamido]-3-[[[2-methyl-5-[[(acetoxy)methoxy]carbonyl]-2H-1,2,4-triazol-3-yl]thio]methyl]-8-oxo-5-thia-1-azabicyclo[4.2.0]oct-2-en-2-carboxylat-acetat sowie von Säureadditionssalzen dieser Verbindung und von Hydraten dieser Verbindung bzw. Säureadditionssalze, dadurch gekennzeichnet, dass man ein entsprechendes Veresterungsmittel einsetzt.

5. Verfahren nach einem der Anprüche 1-3, dadurch gekennzeichnet, dass man als veresterndes Mittel ein Pivaloyloxymethylhalogenid, insbesondere das Jodid, verwendet.